(19) Europäisches Patentamt
European Patent Office
Office européen des brevets

(11) Numéro de publication: **0 210 909 B1**

# (12) FASCICULE DE BREVET EUROPEEN

(45) Date de publication du fascicule du brevet: **21.03.90**

(51) Int. Cl.⁴: **C07C 233/00**, C07D 209/88, A61K 31/16, A61K 31/40

(21) Numéro de dépôt: **86401529.2**

(22) Date de dépôt: **09.07.86**

(54) **Nouveaux alcoylamino amides et leurs sels, leur préparation, leur application comme médicaments et les compositions les renfermant.**

(30) Priorité: **15.07.85 GB 8517854**

(43) Date de publication de la demande: **04.02.87 Bulletin 87/6**

(45) Mention de la délivrance du brevet: **21.03.90 Bulletin 90/12**

(84) Etats contractants désignés: **CH DE FR IT LI NL**

(56) Documents cités:
**EP-A- 0 004 342**
**BE-A- 885 303**
**DE-A- 1 912 849**
**DE-A- 2 655 195**
**DE-A- 2 728 872**
**DE-B- 1 232 159**
**FR-A- 907 128**
**FR-A- 1 051 831**
**FR-M- 4 368**
**GB-A- 764 958**
**US-A- 2 499 352**

**BERICHTE DER DEUTSCHEN CHEMISCHEN GESELLSCHAFT, vol. 60, 1927, pages 345-358, Berlin; J.U. BRAUN et al. "Decarboxypeptide und ihre Derivate (I)"**

(73) Titulaire: **ROUSSEL-UCLAF, 35, boulevard des Invalides, F-75007 Paris(FR)**

(72) Inventeur: **Kay, David Paul, 4 Church Path - Purton, Swindon Wiltshire(GB)**
Inventeur: **Kennewell, Peter David, 10 St Helen's View Okus, Swindon Wiltshire(GB)**

(74) Mandataire: **Vieillefosse, Jean-Claude et al, Département des Brevets ROUSSEL UCLAF B.P no 9, F-93230 Romainville(FR)**

(56) Documents cités: (suite)
**BEILSTEINS HANDBUCH DER ORGANISCHEN CHEMIE, vol. 12, 4eme édition, page 1068, Verlag von J. Springer, Berlin, 1929; "Isocyclische Reihe Monoamine"**

**EP 0 210 909 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention concerne de nouveaux alcoylamino amides et leurs sels, ainsi que le procédé de préparation, l'application à titre de médicaments de ces nouveaux produits et les compositions pharmaceutiques les renfermant.

Les brevets français FR-A 907 128, FR-A 1 051 831, anglais GB-A 764 958 ou allemands DE-B 1 232 159, DE-A 2 728 872, DE-A 1 912 849 ou DE-A 2 655 195, notamment, décrivent des produits présentant des groupements alcoylamino-amides. Ces produits sont le plus souvent présentés comme possédant des propriétés anesthériques et ou analgésiques.

L'invention a pour objet de nouveaux alcoylamino amides ainsi que leurs sels d'addition avec les acides, caractérisés en ce qu'ils répondent à la formule:

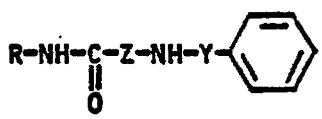

(I)

dans laquelle R représente un groupe de formule:

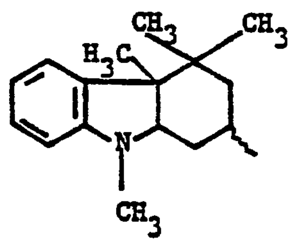

,

Z représente un radical $-(CH_2)_n-$ éventuellement substitué par un radical alcoyle renfermant de 1 à 6 atomes de carbone et dans lequel n est un nombre entier qui varie de 1 à 6 et Y représente un groupe de formule

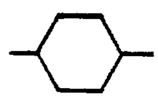

ou représente un radical $-(CH_2)_n-$ dans lequel n est un nombre entier qui varie de 1 à 6.

Dans la formule (I) et dans ce qui suit, le terme radical alcoyle renfermant de 1 à 8 atomes de carbone désigne par exemple un radical méthyle, éthyle, propyle, isopropyle, butyle, pentyle, hexyle, heptyle ou octyle.

Les produits de formule (I) présentent un caractère basique; ils peuvent par conséquent former des sels d'addition avec les acides tels que par exemple les sels formés avec les acides chlorhydrique, bromhydrique, iodhydrique, nitrique, sulfurique, phosphorique, acétique, formique, benzoïque, maléique, fumarique, succinique, tartrique, citrique, oxalique, glyoxylique, aspartique, alcanesulfoniques, tels que l'acide méthanesulfonique et arylsulfoniques, tels que l'acide benzènesulfonique.

Parmi les produits objet de l'invention, on peut citer notamment les dérivés répondant à la formule (I) ci-dessus, ainsi que leurs sels d'addition avec les acides caractérisés en ce que dans ladite formule (I), R représente un groupement (1,2,3,4,4a,9a-hexahydro-4,4,4a,9-tétraméthylcarbazol-2α-yl), un groupement (1,2,3,4,4a,9a-hexahydro-4,4,4a,9a-tétraméthylcarbazol-2β-yl),

Z représente un radical $-CH_2-$, $-(CH_2)_2-$ ou $-(CH_2)_3-$ et Y représente un groupe de formule:

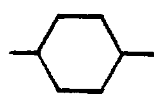

ou un radical $-(CH_2)_3$ ou $(CH_2)_4$.

Parmi les produits objet de l'invention, on retient plus particulièrement les dérivés répondant à la formule (I) ci-dessus, ainsi que leurs sels d'addition avec les acides, caractérisés en ce que R représente un groupement (1,2,3,4,4a,9a-hexahydro-4,4,4a,9-tétraméthylcarbazol-2α-yl), un groupement (1,2,3,4,4a,9a-hexahydro-4,4,4a,9-tétraméthylcarbazol-2β-yl),

Z représente un radical $-(CH_2)_2-$ et Y représente un groupe de formule:

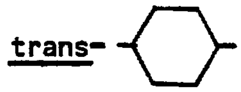

ou un radical $-(CH_2)_4-$.

Parmi ces derniers, on peut citer tout particulièrement les dérivés dont les noms suivent:

- N-(1,2,3,4,4a,9a-hexahydro-4,4,4a,9-tétraméthylcarbazol-2β-yl)-3-(trans-4-phénylcyclohexylamino) propionamide;
- N-(1,2,3,4,4a,9a-hexahydro-4,4,4a,9-tétraméthylcarbazol-2β-yl)-2-(trans-4-phénylcyclohexylamino) acétamide;
- N-(1,2,3,4,4a,9a-hexahydro-4,4,4a,9-tétraméthylcarbazol-2α-yl)-3-(4-phénylbutylamino) propionamide;
- N-(1,2,3,4,4a,9a-hexahydro-4,4,4a,9-tétraméthylcarbazol-2β-yl)-3-(3-phénylpropylamino) propionamide;
- N-(1,2,3,4,4a,9a-hexahydro-4,4,4a,9-tétraméthylcarbazol-2α-yl)-2-(4-phénylbutylamino) acétamide;

ainsi que leurs sels d'addition avec les acides.

L'invention a également pour objet un procédé de préparation de nouveaux alcoylamino amides tels que définis par la formule (I) ci-dessus, ainsi que de leurs sels d'addition avec les acides, caractérisé en ce que l'on fait réagir une amine de formule:

$$R{-}NH_2 \quad (II)$$

dans laquelle R a la signification déjà indiquée avec un produit de formule:

$$Cl{-}CO{-}Z{-}Hal \quad (III)$$

dans laquelle Z a la signification déjà indiquée et Hal représente un atome de chlore ou de brome, en présence d'un agent basique, pour obtenir un produit de formule:

$$R{-}NH{-}CO{-}Z{-}Hal \quad (IV)$$

dans laquelle R, Z et Hal ont la signification déjà indiquée, produit de formule (IV) que l'on fait réagir avec un produit de formule:

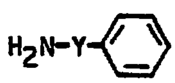

(V)

dans laquelle Y a la signification déjà indiquée pour obtenir un produit de formule (I) correspondant que l'on isole et si désiré, salifie.

Dans des conditions préférentielles de mise en œuvre de l'invention, le procédé de préparation ci-dessus décrit est caractérisé en ce que:

a) la réaction du produit de formule (II) avec le produit de formule (III) est effectuée en présence d'un agent basique tel que le carbonate de potassium au sein d'un solvant organique tel que le dichlorométhane,

b) la réaction du produit de formule (IV) avec le produit de formule (V) est effectuée au sein d'un solvant organique aromatique comme par exemple le benzène ou le toluène.

Les sels d'addition des produits de formule (I) peuvent être préparés en faisant réagir, en proportions sensiblement stoéchiométriques, un acide minéral ou organique avec lesdits produits de formule (I). Les sels peuvent être préparés sans isoler les bases correspondantes.

Les produits, objet de la présente invention, possèdent de très intéressantes propriétés pharmacologiques; ils sont doués notamment de remarquables propriétés anti-oedemateuses et anti-inflammatoires comme le montre le test de l'oedème provoqué par la carraghemine, décrit dans la partie expérimentale.

Ces propriétés justifient l'utilisation des nouveaux dérivés d'alcoylamino amides, objet de la présente invention, ainsi que de leurs sels d'addition avec les acides pharmaceutiquement acceptables à titre de médicaments.

La présente demande a ainsi également pour objet l'application à titre de médicaments des nouveaux dérivés alcoylamino amides tels que définis par la formule (I) ainsi que de leurs sels d'addition avec les acides pharmaceutiquement acceptables.

Parmi les médicaments objet de l'invention, on retient de préférence les médicaments, caractérisés en ce qu'ils sont constitués par les dérivés répondant à la formule (I) dans laquelle

R représente un groupement (1,2,3,4,4a,9a-hexahydro-4,4,4a,9-tétraméthylcarbazol-2α-yl), un groupement (1,2,3,4,4a,9a-hexahydro-4,4,4a,9a-tétraméthylcarbazol-2β-yl),

Z représente un radical $-CH_2-$, $-(CH_2)_2-$ ou $-(CH_2)_3-$ et Y représente un groupe de formule:

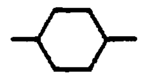

ou un radical $-(CH_2)_3$ ou $(CH_2)_4$,

ainsi que par leurs sels d'addition avec les acides pharmaceutiquement acceptables.

Parmi ceux-ci, on retient notamment les médicaments caractérisés en ce qu'ils sont constitués par les dérivés répondant à la formule (I) dans laquelle R représente un groupement (1,2,3,4,4a,9a-hexahydro-4,4,4a,9-tétraméthylcarbazol-2α-yl), un groupement (1,2,3,4,4a,9a-hexahydro-4,4,4a,9-tétramethyl-carbazol-2β-yl), Z représente un radical $-(CH_2)_2-$ et Y représente un groupe de formule:

<u>trans</u>- 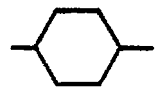

ou un radical $-(CH_2)_4-$,

ainsi que par leurs sels d'addition avec les acides pharmaceutiquement acceptables.

Parmi ces derniers, on retient tout particulièrement les produits dont les noms suivent:

- N-(1,2,3,4,4a,9a-hexahydro-4,4,4a,9-tétraméthylcarbazol-2β-yl)-3-(<u>trans</u>-4-phénylcyclohexyl-amino) propionamide;
- N-(1,2,3,4,4a,9a-hexahydro-4,4,4a,9-tétraméthylcarbazol-2β-yl)-2-(<u>trans</u>-4-phénylcyclohexyl-amino) acétamide;
- N-(1,2,3,4,4a,9a-hexahydro-4,4,4a,9-tétraméthylcarbazol-2α-yl)-3-(4-phénylbutylamino) propionamide;
- N-(1,2,3,4,4a,9a-hexahydro-4,4,4a,9-tétraméthylcarbazol-2β-yl)-3-(3-phénylpropylamino) propionamide;
- N-(1,2,3,4,4a,9a-hexahydro-4,4,4a,9-tétraméthylcarbazol-2α-yl)-2-(4-phénylbutylamino) acétamide;

ainsi que leurs sels d'addition avec les acides pharmaceutiquement acceptables.

Ces médicaments trouvent par exemple leur emploi dans le traitement des troubles inflammatoires.

La dose usuelle variable selon le produit utilisé, le sujet traité et l'affection en cause peut être, par exemple, de 0,10 mg à 100 mg par jour, et de préférence de 1 mg à 20 mg par jour par voie orale chez l'homme.

L'invention a ainsi pour objet les compositions pharmaceutiques qui renferment au moins un dérivé précité ou l'un de ses sels d'addition avec les acides pharmaceutiquement acceptables, à titre de principe actif.

A titre de médicaments, les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être par exemple solides ou liquides et se présenter sous les formes pharmaceutiques couramment utilisées en médecine humaine, comme par exemple, les comprimés, simples ou dragéifiés, les gélules, les granulés, les suppositoires, les sirops, les solutions, les préparations injectables; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les amines de formule (II) sont des produits connus d'une façon générale. Toutefois, le 2-amino 1,2,3,4,4a,9a-hexahydro-4,4,4a,9-tétraméthylcarbazole est un produit nouveau. Il peut être préparé par action du cyanoborohydrure de sodium sur la 3,4,4a,9a-tétrahydro-4,4,4a,9-tétraméthylcarbazol-2(1H)-one préparée comme indiqué dans J. Chem. Soc. 1955, 4369 en présence d'un ammonium tel que l'acétate d'ammonium.

Un exemple d'une telle préparation est donnée dans la partie expérimentale.

L'invention a donc ainsi pour objet à titre de produit industriel nouveau utile notamment à la préparation des produits de formule (I), le 2-amino 1,2,3,4,4a,9a-hexahydro-4,4,4a,9-tétraméthylcarbazole.

Les exemples suivants illustrent l'invention sans toutefois la limiter.

Préparation du 2-amino 1,2,3,4,4a,9a-hexahydro-4;4,4a,9-tétraméthylcarbazole.

On mélange sous atmosphère inerte, à température ambiante pendant 16 heures, 22 g de 3,4,4a,9a-tétrahydro-4,4,4a,9-tétraméthylcarbazol-2(1H)-one préparé comme indiqué dans J. Chem. Soc. 1955, 4369, 37 g d'acétate d'ammonium et 4 g de cyanoborohydrure de sodium dans 300 cm3 de méthanol. On

concentre jusqu'à un volume de 70 cm3, dilue à l'eau, acidifie jusqu'à pH 1 à l'aide d'acide chlorhydrique concentré et agite 1 heure à température ambiante. On extrait le milieu réactionnel à l'éther, ajuste à pH 5 à l'aide d'ammoniaque concentré, extrait de nouveau à l'éther, alcalinise jusqu'à pH 10 à l'aide d'ammoniaque concentré et extrait à l'éther. On récupère 11 g de produit attendu sous forme d'un mélange d'isomères α et β-aminés.

Exemple 1: N-(1,2,3,4,4a,9a-hexahydro-4,4,4a,9-tétraméthylcarbazol-2β-yl)-3-(trans-4-phénylcyclohexylamino) propionamide et son dichlorhydrate.

Stade A: N-(1,2,3,4,4a,9a-hexahydro-4,4,4a,9-tétraméthylcarbazol-2β-yl)-3-chloropropionamide.

On ajoute peu à peu 14 g de chlorure de 3-chloropropionyle dans un mélange comprenant 24,4 g de 2-amino 1,2,3,4,4a,9a-hexahydro-4,4,4a,9-tétraméthylcarbazole et 25 g de carbonate de potassium dans 300 cm3 de dichlorométhane et maintient 16 heures sous agitation. On lave la phase organique à l'acide chlorhydrique 2N puis avec une solution aqueuse de bicarbonate de soude, sèche et élimine les solvants sous pression réduite. On chromatographie le résidu sur silice en éluant au dichlorométhane et obtient 6 g de produit attendu F = 168–170°C et 12 g d'isomère 2 F = 166–168°C.

Spectre IR:

Isomère 2β: 3260 $^{cm-1}$, 1630 $^{cm-1}$
Isomère 2α: 3215 $^{cm-1}$, 1630 $^{cm-1}$.

Stade B: N-(1,2,3,4,4a,9a-hexahydro-4,4,4a,9-tétraméthylcarbazol-2β-yl)-3-(trans-4-phénylcyclohexylamino) propionamide et son dichlorhydrate.

On chauffe au reflux dans du toluène pendant 3 jours 1,5 g de produit obtenu au stade A et 3 g de trans-4-phénylcyclohexylamine préparé comme dans J. Org. Chem. (1952) 17, 1017. On refroidit le milieu réactionnel, filtre et élimine le solvant sous pression réduite. On chromatographie le résidu sur silice (éluant:dichlorométhane-méthanol 95-5) et obtient 1,36 g de produit attendu sous forme de base que l'on dissout dans du méthanol et traite à l'aide d'une solution d'acide chlorhydrique dans l'éther pour obtenir le dichlorhydrate. F = 184–186°C.

Spectre IR:

3410 $^{cm-1}$, 3265 $^{cm-1}$, 1680 $^{cm-1}$.

Exemples 2 à 10:

En utilisant une méthode analogue à celle utilisée à l'exemple 1, mais en partant des amines II et V et des chlorures d'acide de formule (III) appropriés, on a préparé les composés des exemples 2 à 10.

Les rendements, points de fusion, résultats de microanalyse et de spectre IR sont donnés dans le tableau I ci-après.

Exemple 2: N-(1,2,3,4,4a,9a-hexahydro-4,4,4a,9-tétraméthylcarbazol-2α-yl)-3-(trans-4-phénylcyclohexylamino) propionamide.

Exemple 3: N-(1,2,3,4,4a,9a-hexahydro-4,4,4a,9-tétraméthylcarbazol-2β-yl)-2-(trans-4-phénylcyclohexylamino) acétamide.

Exemple 4: N-(1,2,3,4,4a,9a-hexahydro-4,4,4a,9-tétraméthylcarbazol-2α-yl)-2-(trans-4-phénylcyclohexylamino) acétamide.

Exemple 5: N-(1,2,3,4,4a,9a-hexahydro-4,4,4a,9-tétraméthylcarbazol-2β-yl)-3-(4-phénylbutylamino) propionamide.

Exemple 6: N-(1,2,3,4,4a,9a-hexahydro-4,4,4a,9-tétraméthylcarbazol-2α-yl)-3-(4-phénylbutylamino) propionamide.

Exemple 7: N-(1,2,3,4,4a,9a-hexahydro-4,4,4a,9-tétraméthylcarbazol-2β-yl)-3-(3-phénylpropylamino) propionamide.

Exemple 8: N-(1,2,3,4,4a,9a-hexahydro-4,4,4a,9-tétraméthylcarbazol-2α-yl)-3-(3-phénylpropylamino) propionamide.

Exemple 9: N-(1,2,3,4,4a,9a-hexahydro-4,4,4a,9-tétraméthylcarbazol-2β-yl)-2-(4-phénylbutylamino) acétamide.

Exemple 10: N-(1,2,3,4,4a,9a-hexahydro-4,4,4a,9-tétraméthylcarbazol-2α-yl)-2-(4-phénylbutylamino) acétamide.

## TABLEAU I

RNHCO-Z-NH-Y-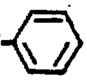

| Ex. | R | Z | Y | % | spectre IR(Kbr) $cm^{-1}$ | F°C |
|---|---|---|---|---|---|---|
| 1 | β 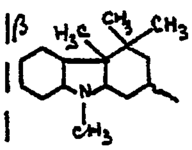 | $(CH_2)_2$ | trans 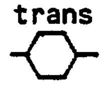 | 66 | 3410,3265,1680,1660 | 184-6° |
| 2 | α " | " | " | 85 | 3390,1650,1600 | résine |
| 3 | β " | $CH_2$ | " | 98 | 3400,3300,1655,1600 | 155-6° |
| 4 | α " | " | " | 70 | 3300,1650 | résine |
| 5 | β " | $(CH_2)_2$ | $(CH_2)_4$ | 60 | 3400,3220,1650 | résine |
| 6 | α " | " | " | 75 | 3280,1640,1600 | résine |
| 7 | β " | " | $(CH_2)_3$ | 64 | 3400,3250,1650 | résine |
| 8 | α " | " | " | 46 | 3280,1640,1600 | résine |
| 9 | β " | $CH_2$ | $(CH_2)_4$ | 54 | 3300,1650,1600 | résine |
| 10 | α " | " | " | 59 | 3320,1660,1640,1600 | résine |

TABLEAU I (suite)

RNHCO-Z-NH-Y-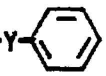

| Ex. | Formule | Poids Mol. | Microanalyse : Calculé/Trouvé | | | | |
|---|---|---|---|---|---|---|---|
| | | | C | H | N | Cl | |
| 2 | $C_{31}H_{43}N_3O.1/2H_2O$ | 482.7 | 77.13<br>77.43 | 9.19<br>9.09 | 8.70<br>8.70 | | |
| 3 | $C_{30}H_{41}N_3O.2HCl$ | 532.6 | 67.66<br>67.93 | 8.14<br>8.12 | 7.89<br>7.85 | | |
| 4 | $C_{30}H_{41}N_3O$ | 459.7 | 78.39<br>77.91 | 8.99<br>9.00 | 9.14<br>9.06 | | |
| 5 | $C_{29}H_{41}N_3O$ | 447.7 | 76.94<br>76.98 | 9.15<br>9.14 | 9.17<br>9.26 | | Théorie + 1,3% $CH_2Cl_2$ |
| 6 | $C_{29}H_{41}N_3O$ | 447.7 | 76.89<br>76.92 | 9.14<br>9.13 | 9.08<br>9.25 | | Théorie + 1,4% $CH_2Cl_2$ |
| 7 | $C_{28}H_{39}N_3O$ | 433.6 | | | | | |
| 8 | $C_{28}H_{39}N_3O$ | 433.6 | | | | | |
| 9 | $C_{28}H_{39}N_3O$ | 433.6 | 77.16<br>77.27 | 9.12<br>9.03 | 9.58<br>9.63 | | Théorie + 0,6% $CH_2Cl_2$ |
| 10 | $C_{28}H_{39}N_3O$ | 433.6 | 76.08<br>76.36 | 8.87<br>8.89 | 9.47<br>9.50 | | Théorie + 2,0% $CH_2Cl_2$ |

Exemple 11:
On a préparé des comprimés répondant à la formule suivante:
– composé de l'exemple 1 20 mg
– excipient q.s. pour un comprimé terminé à 150 mg.
(détail de l'excipient: lactose, amidon, talc, magnésium de stéarate).
Exemple 12:
On a préparé des comprimés répondant à la formule suivante:
– composé de l'exemple 6 20 mg
– excipient q.s. pour un comprimé terminé à 150 mg.
(détail de l'excipient: lactose, amidon, talc, magnésium de stéarate).

ETUDE PHARMACOLOGIQUE

Les propriétés anti-oedémateuses des composés de formule (I) ont été déterminées en effectuant une variante du test de l'oedème provoqué par la carraghenine chez le rat mâle Wistar (Proc. Soc. Exp. Biol. Med. 1962, 11, 544).
Les résultats notés dans le tableau II indiquent le pourcentage d'inhibition en poids, par rapport aux témoins après administration de 100 mg/kg per os ou de 50 mg/kg par voie intrapéritonéale.

**TABLEAU II**

| Exemple | Inhibition (%) |
|---|---|
| 1 | 87,7 (i.p.), 14,1 (p.o.) |
| 2 | 82,3 (i.p.), 34,8 (p.o.) |
| 3 | 57,4 (i.p.), 12,9 (p.o.) |
| 5 | 36,4 (p.o.) |
| 6 | 43,6 (p.o.) |
| 7 | 16,5 (p.o.) |
| 8 | 40,8 (p.o.) |
| 9 | 28,7 (p.o.) |
| 10 | 15,1 (p.o.) |

**Revendications**

1. Alcoylamino amides et leurs sels d'addition avec les acides, caractérisés en ce qu'ils répondent à la formule:

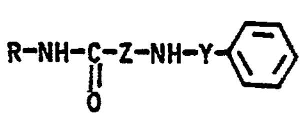

(I)

dans laquelle R représente un groupe de formule:

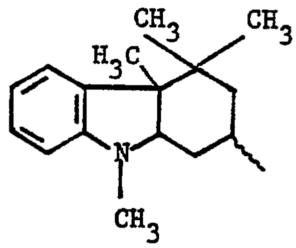

,

Z représente un radical $-(CH_2)_n-$ éventuellement substitué par un radical alcoyle renfermant de 1 à 6 atomes de carbone et dans lequel n est un nombre entier qui varie de 1 à 6, Y représente un groupe de formule

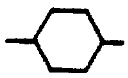

ou représente un radical $-(CH_2)_n-$ dans lequel n est un nombre entier qui varie de 1 à 6.

2. Dérivés répondant à la formule générale (I) de la revendication 1, ainsi que leurs sels, caractérisés en ce que dans ladite formule (I), R représente un groupement (1,2,3,4,4a,9a-hexahydro-4,4,4a,9-tétraméthylcarbazol-2α-yl), un groupement (1,2,3,4,4a,9a-hexahydro-4,4,4a,9a-tétraméthylcarbazol-2β-yl),

Z représente un radical $-CH_2-$, $-(CH_2)_2-$ ou $-(CH_2)_3-$ et Y représente un groupe de formule:

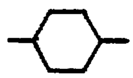

ou un radical $-(CH_2)_3$ ou $(CH_2)_4$.

3. Dérivés répondant à la formule générale (I) de la revendication 1, ainsi que leurs sels, caractérisés en ce que dans ladite formule (I), R représente un groupement (1,2,3,4,4a,9a-hexahydro-4,4,4a,9-tétraméthylcarbazol-2α-yl), un groupement (1,2,3,4,4a,9a-hexahydro-4,4,4a,9-tétraméthylcarbazol-2β-yl),

Z représente un radical $-(CH_2)_2-$ et Y représente un groupe de formule:

trans- 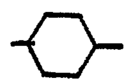

ou un radical $-(CH_2)_4-$.

4. L'un quelconque des dérivés de formule (I) telle que définie à la revendication 1, dont les noms suivent:

- N-(1,2,3,4,4a,9a-hexahydro-4,4,4a,9-tétraméthylcarbazol-2β-yl)-3-(trans-4-phénylcyclohexylamino) propionamide;
- N-(1,2,3,4,4a,9a-hexahydro-4,4,4a,9-tétraméthylcarbazol-2β-yl)-2-(trans-4-phénylcyclohexylamino) acétamide;
- N-(1,2,3,4,4a,9a-hexahydro-4,4,4a,9-tétraméthylcarbazol-2α-yl)-3-(4-phénylbutylamino) propionamide;
- N-(1,2,3,4,4a,9a-hexahydro-4,4,4a,9-tétraméthylcarbazol-2β-yl)-3-(3-phénylpropylamino) propionamide;
- N-(1,2,3,4,4a,9a-hexahydro-4,4,4a,9-tétraméthylcarbazol-2α-yl)-2-(4-phénylbutylamino) acétamide;

ainsi que leurs sels d'addition avec les acides.

5. Procédé de préparation des nouveaux alcoylamino amides tels que définis par la formule (I) de la revendication 1, ainsi que de leurs sels d'addition avec les acides caractérisé en ce que l'on fait réagir une amine de formule

$$R{-}NH_2 \qquad (II)$$

dans laquelle R a la signification déjà indiquée avec un produit de formule:

$$Cl{-}CO{-}Z{-}Hal \qquad (III)$$

dans laquelle Z a la signification déjà indiquée et Hal représente un atome de chlore ou de brome, en présence d'un agent basique, pour obtenir un produit de formule:

$$R{-}NH{-}CO{-}Z{-}Hal \qquad (IV)$$

dans laquelle R, Z et Hal ont la signification déjà indiquée, produit de formule (IV) que l'on fait réagir avec un produit de formule:

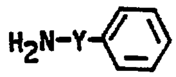

(V)

dans laquelle Y a la signification déjà indiquée pour obtenir un produit de formule (I) correspondant que l'on isole et si désiré, salifie.

6. Procédé selon la revendication 5, caractérisé en ce que:

a) la réaction du produit de formule (II) avec le produit de formule (III) est effectuée en présence d'un agent basique tel que le carbonate de potassium au sein d'un solvant organique tel que le dichlorométhane,

b) la réaction du produit de formule (IV) avec le produit de formule (V) est effectuée au sein d'un solvant organique aromatique tel que le benzène ou le toluène.

7. Médicaments caractérisés en ce qu'ils sont constitués par les nouveaux alcoylamino amides tels que définis par la formule (I) de la revendication 1, ainsi que par leurs sels d'addition avec les acides pharmaceutiquement acceptables.

8. Médicaments caractérisés en ce qu'ils sont constitués par les nouveaux dérivés tels que définis à la revendication 2 ou 3, ainsi que par leurs sels d'addition avec les acides pharmaceutiquement acceptables.

9. Médicaments caractérisés en ce qu'ils sont constitués par les nouveaux dérivés tels que définis à la revendication 4, ainsi que par leurs sels d'addition avec les acides pharmaceutiquement acceptables.

10. Compositions pharmaceutiques, caractérisées en ce qu'elles renferment à titre de principe actif, l'un au moins des médicaments tels que définis à l'une quelconque des revendications 7, 8 ou 9.

11. A titre de produit industriel le 2-amino 1,2,3,4,4a,9a-hexahydro-4,4,4a,9-tétraméthylcarbazole.

**Claims**

1. New alkylamino amides and their addition salts with acids, characterized in that they correspond to the formula:

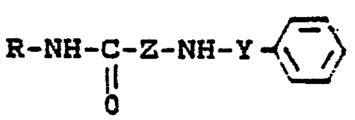

(I)

in which R represents a group of the formula:

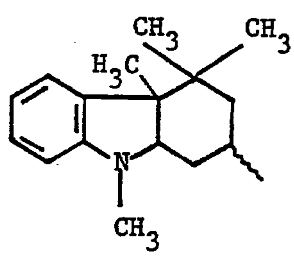

Z represents a $-(CH_2)_n-$ radical, optionally substituted by an alkyl radical containing 1 to 6 carbon atoms, and in which n is an integer which varies from 1 to 6, Y represents a group of the formula

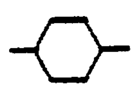

or represents a $-(CH_2)_n-$ radical in which n is an integer which varies from 1 to 6.

2. Derivatives according to the general formula (I) of claim 1, and also their salts, characterized in that in the said formula (I), R represents a (1,2,3,4,4a,9a-hexahydro-4,4,4a,9-tetramethylcarbazole-2alpha-yl) group or a (1,2,3,4,4a,9a-hexahydro-4,4,4a,9-tetramethylcarbazole-2beta-yl) group, Z represents a $-CH_2-$, $-(CH_2)_2-$ or $-(CH_2)_3-$ radical and Y represents a group of the formula:

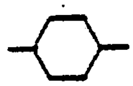

or a $-(CH_2)_3$ or $(CH_2)_4$ radical.

3. Derivatives according to the general formula (I) of claim 1, and also their salts, characterized in that in the said formula (I), R represents a (1,2,3,4,4a,9a-hexahydro-4,4,4a,9-tetramethylcarbazole-2alpha-yl) group, or a (1,2,3,4,4a,9a-hexahydro-4,4,4a,9-tetramethylcarbazole-2beta-yl) group, Z represents a $-(CH_2)_2-$ radical and Y represents a group of the formula:

trans 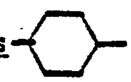

or a $-(CH_2)_4-$ radical.

4. Any of the derivatives of formula (I) as defined in claim 1 the names of which follow:
- N-(1,2,3,4,4a,9a-hexahydro-4,4,4a,9-tetramethylcarbazole-2beta-yl)-3-(trans-4-phenylcyclohexylamino) propionamide;
- N-(1,2,3,4,4a,9a-hexahydro-4,4,4a,9-tetramethylcarbazole-2beta-yl)-2-(trans-4-phenylcyclohexylamino) acetamide;
- N-(1,2,3,4,4a,9a-hexahydro-4,4,4a,9-tetramethylcarbazole-2alpha-yl)-3-(4-phenylbutylamino) propionamide;
- N-(1,2,3,4,4a,9a-hexahydro-4,4,4a,9-tetramethylcarbazole-2beta-yl)-3-(3-phenylpropylamino) propionamide;
- N-(1,2,3,4,4a,9a-hexahydro-4,4,4a,9-tetramethylcarbazole-2alpha-yl)-2-(4-phenylbutylamino) acetamide;

and their addition salts with acids.

5. Process for the preparation of the new alkylamino amides as defined by formula (I) of claim 1, and also their addition salts with acids, characterized in that an amine of the formula:

$$R-NH_2 \qquad (II)$$

in which R has the meaning already indicated, is reacted with a product of the formula:

$$Cl-CO-Z-Hal \qquad (III)$$

in which Z has the meaning already indicated and Hal represents a chlorine or bromine atom, in the presence of a basic agent, to obtain a product of formula:

$$R-NH-CO-Z-Hal \qquad (IV)$$

in which R, Z and Hal have the meaning already indicated, which product of formula (IV) is reacted with a product of formula:

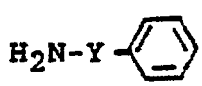 (V)

in which Y has the meaning already indicated, to obtain a corresponding product of formula (I) which is isolated and, if desired, salified.

6. Process according to claim 5, characterized in that:
a) the reaction of the product of formula (II) with the product of formula (III) is carried out in the presence of a basic agent such as potassium carbonate in an organic solvent such as dichloromethane,
b) the reaction of the product of formula (IV) with the product of formula (V) is carried out in an aromatic organic solvent such as benzene or toluene.

7. Medicaments, characterized in that they are composed of the new alkylamino amides as defined by formula (I) of claim 1, and also of their addition salts with pharmaceutically acceptable acids.

8. Medicaments, characterized in that they are composed of the new derivatives such as defined in claim 2 or 3, and also of their addition salts with pharmaceutically acceptable acids.

9. Medicaments characterized in that they are composed of the new derivatives as defined in claim 4, and also of their addition salts with pharmaceutically acceptable acids.

10. Pharmaceutical compositions, characterized, in that they contain as the active principle at least one of the medicaments as defined in any one of claims 7, 8 or 9.

11. As an industrial product, 2-amino 1,2,3,4,4a,9a-hexahydro-4,4,4a,9-tetramethylcarbazole.

## Patentansprüche

1. Neue Alkylaminoamide und deren Additionssalze mit Säuren, dadurch gekennzeichnet, daß sie der Formel

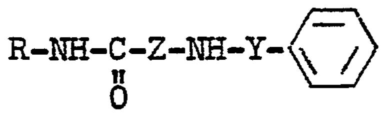

(I)

entsprechen, worin R für eine Gruppe der Formel

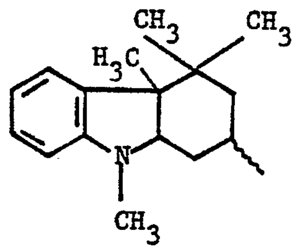

steht; Z einen Rest $-(CH_2)_n-$ wiedergibt, der gegebenenfalls durch einen Alkylrest mit 1 bis 6 Kohlenstoffatomen substituiert ist und in dem n eine ganze Zahl von 1 bis 6 bedeutet; Y eine Gruppe der Formel

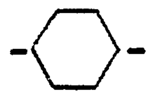

oder einen Rest $-(CH_2)_n-$ wiedergibt, worin n eine ganze Zahl von 1 bis 6 darstellt.

2. Derivate der allgemeinen Formel (I) gemäß Anspruch 1 sowie deren Salze, dadurch gekennzeichnet, daß in der Formel (I) R eine (1,2,3,4,4a,9a-Hexahydro-4,4,4a,9-tetramethylcarbazol-2α-yl)-Gruppe oder eine (1,2,3,4,4a,9a-Hexahydro-4,4,4,9a-tetramethylcarbazol-2β-yl)-Gruppe bedeutet; Z einen Rest $-CH_2-$, $-(CH_2)_2-$ oder $-(CH_2)_3-$ wiedergibt; und Y eine Gruppe der Formel

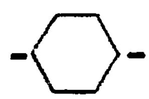

oder einen Rest $-(CH_2)_3$ oder $(CH_2)_4$ darstellt.

3. Derivate der allgemeinen Formel (I) gemäß Anspruch 1 sowie deren Salze, dadurch gekennzeichnet, daß in der Formel (I) R eine (1,2,3,4,4a,9a-Hexahydro-4,4,4a,9-tetramethylcarbazol-2α-yl)-Gruppe oder eine (1,2,3,4,4a,9a-Hexahydro-4,4,4a,9-tetramethylcarbazol-2β-yl)-Gruppe bedeutet; Z einen Rest $-(CH_2)_2-$ wiedergibt; und Y für eine Gruppe der Formel

trans-
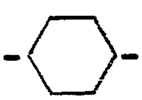

oder einen Rest $-(CH_2)_4-$ steht.

4. Eines der Derivate der Formel (I) gemäß Anspruch 1 mit den folgenden Bezeichnungen:
N-(1,2,3,4,4a,9a-Hexahydro-4,4,4a,9-tetramethylcarbazol-2β-yl)-3-(trans-4-phenylcyclohexylamino)-propionamid;
N-(1,2,3,4,4a,9a-Hexahydro-4,4,4a,9-tetramethylcarbazol-2β-yl)-2-(trans-4-phenylcyclohexylamino)-acetamid;
N-(1,2,3,4,4a,9a-Hexahydro-4,4,4a,9-tetramethylcarbazol-2α-yl)-3-(4-phenylbutylamino)-propionamid;
N-(1,2,3,4,4a,9a-Hexahydro-4,4,4a,9-tetramethylcarbazol-2β-yl)-3-(3-phenylpropylamino)-propionamid;
N-(1,2,3,4,4a,9a-Hexahydro-4,4,4a,9-tetramethylcarbazol-2α-yl)-2-(4-phenylbutylamino)-acetamid;
sowie deren Additionssalze mit Säuren.

5. Verfahren zur Herstellung der neuen Alkylaminoamide der Formel (I) gemäß Anspruch 1 sowie von deren Additionssalzen mit Säuren, dadurch gekennzeichnet, daß man ein Amin der Formel

$$R{-}NH_2 \qquad (II)$$

worin R die angegebene Bedeutung besitzt, mit einem Produkt der Formel

$$Cl{-}CO{-}Z{-}Hal \qquad (III)$$

worin Z die angegebene Bedeutung besitzt und Hal ein Chlor- oder Bromatom wiedergibt, in Gegenwart eines basischen Mittels umsetzt, um zu einem Produkt der Formel

$$R{-}NH{-}CO{-}Z{-}Hal \qquad (IV)$$

zu gelangen, worin R, Z und Hal die angegebene Bedeutung besitzen, dieses Produkt der Formel (IV) mit einem Produkt der Formel

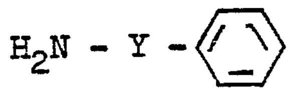

(V)

worin Y die angegebene Bedeutung besitzt, umsetzt, um zu einem entsprechenden Produkt der Formel (I) zu gelangen, das man isoliert und gewünschtenfalls in ein Salz überführt.

6. Verfahren gemäß Anspruch 5, dadurch gekennzeichnet, daß
(a) die Umsetzung des Produkts der Formel (II) mit dem Produkt der Formel (III) in Gegenwart eines basischen Mittels, wie Kaliumcarbonat, in einem organischen Lösungsmittel, wie Methylenchlorid, erfolgt;
(b) die Umsetzung des Produkts der Formel (IV) mit dem Produkt der Formel (V) in einem organischen, aromatischen Lösungsmittel, wie Benzol oder Toluol, durchgeführt wird.

7. Arzneimittel, dadurch gekennzeichnet, daß sie aus den neuen Alkylaminoamiden der Formel (I) gemäß Anspruch 1 sowie aus deren Additionssalzen mit pharmazeutisch verträglichen Säuren bestehen.

8. Arzneimittel, dadurch gekennzeichnet, daß sie aus den neuen Derivaten gemäß Anspruch 2 oder 3 sowie aus deren Additionssalzen mit pharmazeutisch verträglichen Säuren bestehen.

9. Arzneimittel, dadurch gekennzeichnet, daß sie aus den neuen Derivaten gemäß Anspruch 4 sowie aus deren Additionssalzen mit pharmazeutisch verträglichen Säuren bestehen.

10. Pharmazeutische Zusammensetzungen, dadurch gekennzeichnet, daß sie als Wirkstoff zumindest eines der Arzneimittel gemäß einem der Ansprüche 7, 8 oder 9 enthalten.

11. Als industrielles Produkt das 2-Amino-1,2,3,4,4a,9a-hexahydro-4,4,4a,9-tetramethylcarbazol.